(19) Europäisches Patentamt / European Patent Office / Office européen des brevets

(11) **EP 4 491 187 A1**

(12) **EUROPEAN PATENT APPLICATION**
published in accordance with Art. 153(4) EPC

(43) Date of publication:
**15.01.2025  Bulletin 2025/03**

(21) Application number: **23791926.1**

(22) Date of filing: **20.04.2023**

(51) International Patent Classification (IPC):
*A61K 33/00* (2006.01)    *A61K 9/14* (2006.01)
*A61M 1/16* (2006.01)    *A61M 1/36* (2006.01)
*A61P 7/00* (2006.01)    *A61P 13/12* (2006.01)
*A61P 39/02* (2006.01)    *B01D 15/00* (2006.01)
*B01D 69/10* (2006.01)    *B01D 69/12* (2006.01)
*B01D 71/02* (2006.01)    *B01J 20/02* (2006.01)
*B01J 20/20* (2006.01)    *B01J 20/30* (2006.01)

(52) Cooperative Patent Classification (CPC):
**A61K 9/14; A61K 33/00; A61M 1/16; A61M 1/36;
A61P 7/00; A61P 13/12; A61P 39/02; B01D 15/00;
B01D 69/10; B01D 69/12; B01D 71/02;
B01J 20/02; B01J 20/20; B01J 20/30**

(86) International application number:
**PCT/JP2023/015821**

(87) International publication number:
**WO 2023/204278 (26.10.2023 Gazette 2023/43)**

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**
Designated Extension States:
**BA**
Designated Validation States:
**KH MA MD TN**

(30) Priority: **21.04.2022  JP 2022070348
13.07.2022  JP 2022112701
11.10.2022  PCT/JP2022/037909
11.10.2022  JP 2022163375**

(71) Applicant: **Murata Manufacturing Co., Ltd.
Nagaokakyo-shi, Kyoto 617-8555 (JP)**

(72) Inventors:
• **KIMURA, Yuki
Nagaokakyo-shi, Kyoto 617-8555 (JP)**

• **KOBAYASHI, Yusuke
Yokohama-shi, Kanagawa 236-0004 (JP)**
• **WAKUI, Hiromichi
Yokohama-shi, Kanagawa 236-0004 (JP)**
• **AZUSHIMA, Kengo
Yokohama-shi, Kanagawa 236-0004 (JP)**
• **TAMURA, Kouichi
Yokohama-shi, Kanagawa 236-0004 (JP)**
• **TAKASE, Hajime
Yokohama-shi, Kanagawa 236-0004 (JP)**

(74) Representative: **Herrmann, Uwe
Lorenz Seidler Gossel
Rechtsanwälte Patentanwälte
Partnerschaft mbB
Widenmayerstraße 23
80538 München (DE)**

(54) **PHARMACEUTICAL COMPOSITION, ADSORPTION METHOD, TREATMENT METHOD AND PREVENTION METHOD**

(57)  One object of the present disclosure is to provide a novel pharmaceutical composition, preferably a pharmaceutical composition capable of adsorbing various disease-causing substances in vivo. A pharmaceutical composition for adsorbing a disease-causing substance by oral administration, the pharmaceutical composition comprising two-dimensional particles having one or plural layers,
the layers comprising a layer body represented by a formula below:

$$M_m X_n$$

wherein M is at least one metal of Group 3, 4, 5, 6, or 7, X is a carbon atom, a nitrogen atom, or a combination thereof, n is not

EP 4 491 187 A1

less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

**Fig. 1** (a)

3a
1a  } 7a
5a
10a

**Fig. 1** (b)

3a
1a  } 7a
5a
Δd
3b
1b  } 7b
5b
10b

2

**Description**

TECHNICAL FIELD

The present disclosure relates to a pharmaceutical composition, an adsorption method, a treatment method, and a prevention method.

BACKGROUND ART

The number of patients with renal disease is on an increasing trend year by year, and according to the statistics of the Japanese Society for Dialysis Therapy, the number of chronic dialysis patients has increased by approximately 17% from 2010 to 2020. When the renal function decreases due to renal disease, the disease-causing substances accumulate in the blood, and as a result, uremia, electrolyte imbalance, autoimmune disease, and the like can be caused. In order to treat such various symptoms associated with renal disease, a hemodialysis type renal function substitute or the like that removes a disease-causing substance to the outside of the body is used.

Non-Patent Document 1 describes an adsorption type blood purifier using cellulose beads on which cetylamine is immobilized as an adsorbing body as a filter used for a hemodialysis type renal function substitute.

On the other hand, as a method for removing a disease-causing substance in place of a renal function substitute, an adsorbent that is orally ingested, adsorbs a toxic substance in the body, and discharges the substance outside the body has been developed.

As such an adsorbent, Non-patent Document 2 describes medicinal charcoal capable of adsorbing gas and poison in the gastrointestinal tract.

Non-Patent Documents 3 and 4 describe carbon-based adsorbents capable of adsorbing uremic toxins present in the gastrointestinal tract without being absorbed in the body and being excreted with feces.

Non-Patent Document 5 describes a calcium type cation exchange resin capable of exchanging potassium ions in the intestinal tract with calcium ions in the structure to reduce the blood potassium value.

Non-Patent Document 6 describes a polycationic polymer that can be directly excreted in feces without being absorbed by binding to phosphate ions released from food in the gastrointestinal tract.

PRIOR ART DOCUMENT

PATENT DOCUMENT

Non-patent Document 1: The package insert of medical devices "Lixelle", KANEKA CORPORATION, revised in January 2017 (10th edition)

Non-patent Document 2: Pharmaceutical Interview Form "medicinal charcoal", Nichi-Iko Pharmaceutical Co., Ltd., revised in January 2019 (4th edition)

Non-Patent Document 3: Pharmaceutical Interview Form "KREMEZIN Tablets 500 mg", Mitsubishi Tanabe Pharma Corporation, revised in September 2018 (3rd edition)

Non-Patent Document 4: Yoshiteru Honda et al., "Study on Adsorption Property of Spherical Adsorption Charcoal (KREMEZIN Active Material)", Hospital Pharmaceutical Co., Ltd., 1997, Vol. 23, No. 3, 219-224.

Non-Patent Document 5: Pharmaceutical Interview Form "Kalimate Powder", "Kalimate Dry Syrup 92.59%", "Kalimate Oral Solution 20%", Kowa Company, Ltd., revised in September 2020 (20th edition)

Non-patent Document 6: Pharmaceutical Interview Form "PHOSBLOCK Tablets 250 mg", Kyowa Kirin Co., Ltd., revised in November 2020 (1st edition)

SUMMARY OF THE INVENTION

PROBLEMS TO BE SOLVED BY THE INVENTION

The adsorbent described in Non-Patent Document 1 is assumed to act in a blood circuit of a hemodialysis machine by extracorporeal circulation. In addition, all of the adsorbents described in Non-Patent Document 2 to 6 target the adsorbates present in the gastrointestinal tract by ingestion, enterohepatic circulation, production via enterobacteria, or the like. Furthermore, the adsorbate described in Non-Patent Document 1 to 6 is limited to certain materials.

One object of the present disclosure is to provide a novel pharmaceutical composition, preferably a pharmaceutical composition capable of adsorbing various disease-causing substances in vivo. It is also an object of the present disclosure to provide a novel method for adsorbing a disease-causing substance, a novel method for treating a disease-causing substance, or a novel method for preventing a disease-causing substance.

SOLUTIONS TO THE PROBLEMS

The pharmaceutical composition of the present disclosure comprises two-dimensional particles having one or plural layers,

wherein the layers including a layer body represented by a formula below:

$$M_m X_n$$

wherein M is at least one metal of Group 3, 4, 5, 6, or 7, X is a carbon atom, a nitrogen atom, or a combination thereof, n is not less than 1 and not more than 4, m is more than n but not more than 5, and
wherein a modifier or terminal T existing on a surface of the layer body, wherein T is at least one selected from the group consisting of a hydroxyl group, a fluorine atom, a chlorine atom, an oxygen atom, and a hydrogen atom, and is used for adsorbing a disease-causing substance in vivo by oral administration.

EFFECTS OF THE INVENTION

The present disclosure provides a novel pharmaceutical composition, and preferably can provide a pharmaceutical composition capable of adsorbing a disease-causing substance in vivo. The present disclosure may also provide a novel adsorption method, a treatment method, or a prevention methods.
The pharmaceutical composition of the present disclosure contains two-dimensional particles having a layer body represented by $M_m X_n$ and T, and can adsorb a substance that can cause a disease (disease-causing substance), and thus is useful for treatment and/or prevention of various diseases.
In the living body, disease-causing substances such as metabolites, waste products, harmful substances, and excessive ionic substances mainly function in two routes of gastrointestinal tract excretion from the bile duct via the duodenum in the form of being contained in bile in the liver as bile excretion, and excretion in urine via glomerular filtration in the kidney. However, for example, for a patient suffering from renal failure, the excretion function of metabolites and the like by the kidneys is not sufficient, and periodic treatment by dialysis therapy (hemodialysis, peritoneal dialysis, and the like) is required, resulting in significant deterioration of QOL.
Although it should not be construed as being limited to a specific theory, since the two-dimensional particles contained in the pharmaceutical composition of the present disclosure have the ability to predominantly adsorb these disease-causing substances and the like, it is expected that when orally administered, the disease-causing substances in the blood can be adsorbed and eliminated from the blood through mutual access between the intestinal tract and the blood vessel while staying in the intestinal tract. In addition, by adsorbing a substance that can be converted into a harmful substance after being absorbed into blood from the intestinal tract, it is expected to adsorb and eliminate a harmful influence on the body in advance. Then, since it is considered that the two-dimensional particles having adsorbed the disease-causing substances are not absorbed from the intestinal tract, it is expected that the two-dimensional particles pass through the gastrointestinal tract and are excreted as they are together with feces. As described above, although a living body originally has two excretion functions of bile excretion and urine excretion, the metabolic pathway by the pharmaceutical composition of the present disclosure can also be regarded as a third metabolic pathway, and is expected to lead to the reduction of treatment related to dialysis therapy in patients with renal failure, for example. In addition, the two-dimensional particles of the present disclosure also are also expected to adsorb disease-causing substances and the like contained in the contents of the diet in the gastrointestinal tract and do not cause intestinal tract absorption.

BRIEF DESCRIPTION OF THE DRAWINGS

[Fig. 1] Figs. 1(a) and 1(b) are schematic cross-sectional views showing MXene particles of a layered material in one embodiment of the present disclosure, in which
Fig. 1(a) shows single-layer MXene particles, and Fig. 1(b) shows multilayer (exemplarily, two layers) MXene particles.

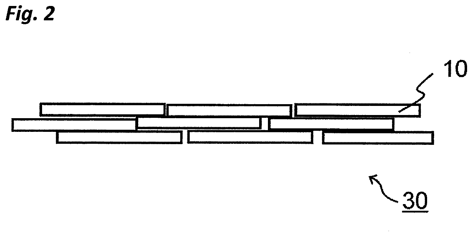

[Fig. 2] Fig. 2 is a schematic cross-sectional view showing a material in one embodiment of the present disclosure.
[Fig. 3] Fig. 3 is a graph showing the results of a MXene administration test to a renal failure model rat, and shows (a) a change in urea nitrogen (BUN) value, (b) a change rate of urea nitrogen (BUN) value, (c) a serum creatinine (Cr) value, (d) a change rate of serum creatinine (Cr) value, and (e) a change in organ weight of the kidney.

[Fig. 4] Fig. 4 shows a result of a MXene administration test to a hypertensive mouse, and shows a behavior area of a mouse in cognitive behavior analysis.

[Fig. 5] Fig. 5 shows a result of a MXene administration test to a hypertensive mouse, and shows a Time of Entries Discrimination Index in cognitive behavior analysis.

DETAILED DESCRIPTION

A pharmaceutical composition of the present disclosure comprises two-dimensional particles of a layered material having one or plural layers,

wherein the layers including a layer body represented by the following composition formula:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

The pharmaceutical composition of the present disclosure contains two-dimensional particles having a layer body represented by $M_mX_n$ and T, and can adsorb a substance that can cause a disease (disease-causing substance), and thus is useful for treatment and/or prevention of various diseases.

In the present disclosure, the layered material may be understood as a layered compound, and the layer is also referred to as "$M_mX_nT_s$". s is an arbitrary number, and conventionally, x or z may be used instead of s. Hereinafter, the layered material may be referred to as MXene, the layer may be referred to as MXene layer, and the two-dimensional particles may be referred to as MXene two-dimensional particle or MXene particles.

In the present disclosure, when an element is referred to as an "atom", the oxidation number of the element is not limited to 0, and may be an arbitrary number within the range of possible oxidation numbers of the element.

In addition, with respect to the reference numerals of the general formulae shown in the present disclosure, unless otherwise specified, definitions for the same reference numerals are common among the general formulae including the reference numerals.

In the above formula: $M_mX_n$, m may typically be, but not limited to, 2, 3, 4, or 5. Also, n may be, but is not limited to, 1, 2, 3, or 4. In one aspect, m may be 3 and n may be 2.

In the above formula: $M_mX_n$, M is preferably at least one selected from the group consisting of Ti, Zr, Hf, V, Nb, Ta, Cr, Mo, Sc, W, and Mn, and more preferably at least one selected from the group consisting of Ti, V, Cr, and Mo.

As $M_mX_n$, one expressed as follows is known.

$Sc_2C$, $Ti_2C$, $Ti_2N$, $Zr_2C$, $Zr_2N$, $Hf_2C$, $Hf_2N$, $V_2C$, $V_2N$, $Nb_2C$, $Ta_2C$, $Cr_2C$, $Cr_2N$, $Mo_2C$, $Mo_{1.3}C$, $Cr_{1.3}C$, $(Ti, V)_2C$, $(Ti, Nb)_2C$, $W_2C$, $W_{1.3}C$, $Mo_2N$, $Nb_{1.3}C$, $Mo_{1.3}Y_{0.6}C$

(In the above formula, "1.3" and "0.6" mean about 1.3 (= 4/3) and about 0.6 (= 2/3), respectively.),

$Ti_3C_2$, $Ti_3N_2$, $Ti_3$ (CN), $Zr_3C_2$, $(Ti, V)_3C_2$, $(Ti_2Nb)C_2$, $(Ti_2Ta)C_2$, $(Ti_2Mn)C_2$, $Hf_3C_2$, $(Hf_2V)C_2$, $(Hf_2Mn)C_2$, $(V_2Ti)C_2$, $(Cr_2Ti)C_2$, $(Cr_2V)C_2$, $(Cr_2Nb)C_2$, $(Cr_2Ta)C_2$, $(Mo_2Sc)C_2$, $(Mo_2Ti)C_2$, $(Mo_2Zr)C_2$, $(Mo_2Hf)C_2$, $(Mo_2V)C_2$, $(Mo_2Nb)C_2$, $(Mo_2Ta)C_2$, $(W_2Ti)C_2$, $(W_2Zr)C_2$, $(W_2Hf)C_2$,

$Ti_4N_3$, $V_4C_3$, $Nb_4C_3$, $Ta_4C_3$, $(Ti, Nb)_4C_3$, $(Nb, Zr)_4C_3$, $(Ti_2Nb_2)C_3$, $(Ti_2Ta_2)C_3$, $(V_2Ti_2)C_3$, $(V_2Nb_2)C_3$, $(V_2Ta_2)C_3$, $(Nb_2Ta_2)C_3$, $(Cr_2Ti_2)C_3$, $(Cr_2V_2)C_3$, $(Cr_2Nb_2)C_3$, $(Cr_2Ta_2)C_3$, $(Mo_2Ti_2)C_3$, $(Mo_2Zr_2)C_3$, $(Mo_2Hf_2)C_3$, $(Mo_2V_2)C_3$, $(Mo_2Nb_2)C_3$, $(Mo_2Ta_2)C_3$, $(W_2Ti_2)C_3$, $(W_2Zr_2)C_3$, $(W_2Hf_2)C_3$, $(Mo_{2.7}V_{1.3})C_3$

(In the above formula, "2.7" and "1.3" mean about 2.7 (= 8/3) and about 1.3 (= 4/3), respectively.)

Typically, in the above formula: $M_mX_n$, M may be Ti or V, X may be a carbon atom or a nitrogen atom, M may be Ti, and X may be a carbon atom. In one aspect, MXene may be $Ti_3C_2T_s$ (In other words, M is Ti, X is C, n is 2, and m is 3.). In this case, a precursor of such MXene (also referred to as a "MAX phase") may be $Ti_3AlC_2$.

MXene can be produced by removing A atoms contained in the MAX phase (In one aspect, the group is represented by $M_mAX_n$, M, m, X, and n have the same meaning as described above, and A is at least one group 12, 13, 14, 15, or 16 element.) of the precursor, but MXene may contain such A atoms. In one aspect, the residual amount of A atoms contained in MXene can be preferably 10 mass% or less, more preferably 8 mass% or less, and still more preferably 6 mass% or less with respect to the content of A atoms in the precursor.

In another embodiment, the residual amount of A atoms may be more than 10 mass%. For example, the two-dimensional particle in which the A atom is removed from only a part of the MAX phase is also included in the technical scope of the two-dimensional particles. Examples of the two-dimensional particle include two-dimensional particles in which A atoms are removed only from the vicinity of the end in the plane direction of the MAX phase (direction parallel to the plane of the $M_mX_n$ layer included in the MAX phase). In this aspect, the residual amount of the A atoms may be, for example, 50 mass% or more, further 80 mass% or more, and particularly 90 mass% or more.

The content of lithium in the two-dimensional particles is preferably not less than 0% by mass and not more than 0.1% by mass, more preferably not less than 0% by mass and not more than 0.01% by mass, and still more preferably not less than 0% by mass and not more than 0.002% by mass. When the content of lithium is within the above range, biocompatibility can be improved.

The content of lithium in the two-dimensional particles can be measured by inductively coupled plasma atomic emission spectrometry (ICP-AES).

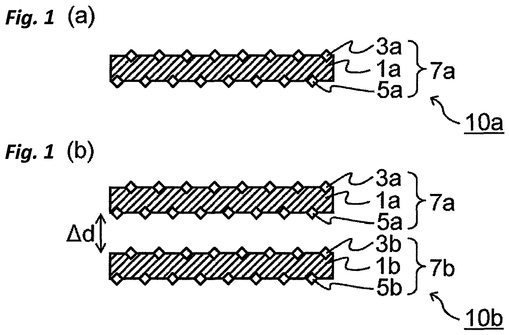

The two-dimensional particles are an aggregate containing MXene particles (hereinafter, simply referred to as "MXene particles") 10a (single-layer MXene particles) of one layer schematically exemplified in Fig. 1(a). Typically, the MXene particle 10a is more specifically the MXene layer 7a having a layer body ($M_mX_n$ layer) 1a represented by $M_mX_n$ and modifier or terminal T3a, 5a presenting on the surface of the layer body 1a (more specifically, at least one of two surfaces facing each other in each layer). Therefore, the MXene layer 7a is also represented as "$M_mX_nT_s$", and s is an arbitrary number.

The two-dimensional particle may include one or plural layers. Examples of the MXene particles (multilayer MXene particles) of the plurality of layers include, but are not limited to, the MXene particle 10b of two layers as schematically shown in Fig. 1(b). 1b, 3b, 5b, and 7b in Fig. 1(b) are the same as 1a, 3a, 5a, and 7a in Fig. 1(a) described above. In the case of the multilayer MXene particles, two adjacent MXene layer (e.g., 7a and 7b) may not necessarily be completely separated from each other, but may be partially in contact with each other. In the single-layer MXene particle 10a, the multilayer MXene particles 10b are individually separated and present in one layer. The MXene may be a mixture of the single-layer MXene particles 10a and the multilayer MXene particles 10b in which unseparated multilayer MXene particles 10b remain.

Typically, at least one of the surfaces of the layer body 1a represented by $M_mX_n$ can be planar (two-dimensional), and all of the surfaces of the layer body 1a can be planar (two-dimensional).

Although the present embodiment is not limited, the thickness of each layer contained in the MXene particles (which corresponds to the MXene layers 7a, 7b) is, for example, not less than 0.8 nm and not more than 5 nm, particularly not less than 0.8 nm and not more than 3 nm (it may vary mainly depending on the number of M atomic layers included in each layer).

The thickness of each layer is determined as a number average dimension (for example, a number average of at least 40) based on an atomic force microscope (AFM) photograph or a transmission electron microscope (TEM) photograph.

For individual laminates of two-dimensional particles (particularly multilayer MXene particles that may be included), the interlayer distance (alternatively, the void dimension is indicated by $\Delta d$ in Fig. 1(b)) may be, for example, 0.8 nm or more and 10 nm or less, particularly not less than 0.8 nm and not more than 5 nm, and more particularly about 1 nm or more, and the total number of layers may be not less than 2 and not more than 20,000.

The interlayer distance in the two-dimensional particle can be measured by obtaining the inter-plane distance (the sum of the interlayer distance and the thickness of each layer) from the position of the peak corresponding to the (002) plane of MXene presenting at $2\theta = 10°$ (deg) or less in the X-ray diffraction measurement of the two-dimensional particle, and subtracting the thickness of each layer from the inter-plane distance.

The two-dimensional particles may contain MXene particles having a small number of layers. The "small number of layers" means that, for example, the number of laminated MXene layers is six or less. In addition, the thickness of the multilayer MXene particle having a small number of layers in the lamination direction is preferably 15 nm or less, and more preferably 10 nm or less. Hereinafter, the "multilayer MXene particles having a small number of layers" may be referred to as "few-layer MXene particles". The single-layer MXene particles and the few-layer MXene particles may be collectively referred to as "single-layer/few-layer MXene particles".

In the two-dimensional particles, the proportion of the single-layer/few-layer MXene particles having a thickness of 15 nm or less may be not less than 0 vol% and not more than 100 vol%, further not less than 0 vol% and not more than 99 vol%, further not less than 0 vol% and not more than 50 vol%, and particularly not less than 0 vol% and not more than 30 vol%.

(Average value of major diameters of two-dimensional surfaces of two-dimensional particles)

The major diameter of the two-dimensional particles are preferably not less than 1 $\mu$m and not more than 20 $\mu$m in a plane (hereinafter, also referred to as a "two-dimensional surface") parallel to each layer. Hereinafter, the average value of the major diameters of the two-dimensional surfaces may be referred to as "average flake size".

The larger the average flake size, the better the orientation of the two-dimensional particles in the material containing the two-dimensional particles. The average value of the major diameters of the two-dimensional surfaces is

preferably 1.5 $\mu$m or more, and more preferably 2.5 $\mu$m or more. When the delamination treatment of MXene is performed by subjecting MXene to the ultrasonic treatment, most of MXene is reduced in diameter to about several hundred nanometers in terms of major diameter by the ultrasonic treatment, so that the membrane formed of the single-layer MXene delaminated by the ultrasonic treatment is considered to have low orientation of two-dimensional particles.

The average value of the major diameters of the two-dimensional surfaces is 20 $\mu$m or less, preferably 15 $\mu$m or less, and more preferably 10 $\mu$m or less from the viewpoint of dispersibility in the dispersion medium.

The major diameter of the two-dimensional surface refers to a major diameter when each MXene particles are approximated to an elliptical shape in an electron microscope photograph obtained by observing the two-dimensional particle from a direction substantially orthogonal to a plane parallel to each layer, and the average value of the major diameters of the two-dimensional surfaces refers to a number average of the major diameters of 80 particles or more. As the electron microscope, a scanning electron microscope (SEM) photograph or a transmission electron microscope (TEM) photograph can be used.

The average value of the major diameters of the two-dimensional particles of the present embodiment may be measured by dissolving a material containing the two-dimensional particles in a solvent and dispersing the two-dimensional particles in the solvent. Alternatively, it may be measured from an SEM image of the material.

(Average value of thicknesses of two-dimensional particles)

The average value of the thicknesses of the two-dimensional particles of the present embodiment is preferably not less than 1 nm and not more than 100 $\mu$m or less. The thickness is preferably 50 $\mu$m or less, and more preferably 20 $\mu$m or less. On the other hand, considering the thickness of the single-layer MXene particles, the lower limit of the thickness of the two-dimensional particles can be 1 nm.

The thickness of the two-dimensional particle can be understood as a length in a direction substantially orthogonal to a plane parallel to each layer, and an average value of the thicknesses of the two-dimensional particles is obtained as a number average dimension (for example, a number average of at least 40 particles) based on an atomic force microscope (AFM) photograph or a transmission electron microscope (TEM) photograph.

The two-dimensional particles can be produced by the following production method, but the two-dimensional particles in the present disclosure are not limited to those produced by the following production method.

In one aspect, the method for producing two-dimensional particles comprises:

(a) providing a precursor represented by the following formula:

$$M_mAX_n$$

wherein M is at least one metal of Group 3, 4, 5, 6, or 7, X is a carbon atom, a nitrogen atom, or a combination thereof, A is at least one group 12, 13, 14, 15, or 16 element, n is not less than 1 and not more than 4, and m is more than n and not more than 5,

(b) removing at least a part of the A atoms from the precursor by etching using an etching liquid to obtain the etched product,

(c) cleaning the etched product to obtain a cleaned product, and

the method for producing two-dimensional particles may further comprise:

(d) performing an intercalation treatment on the etched product in a dispersion medium using a metal-containing compound to obtain an intercalated product; and

(e) performing a delamination treatment on the intercalated product to obtain a delaminated product.

In one aspect, the etched product and the delaminated product can be used as the two-dimensional particles, and preferably the cleaned product can be used as the two-dimensional particles.

Each step is described below.

• Step (a)

First, a predetermined precursor is prepared. The predetermined precursor that can be used in the present embodiment is a MAX phase that is a precursor of MXene, and represented by

the following formula:

$$M_mAX_n$$

wherein M is at least one metal of Group 3, 4, 5, 6, or 7, X is a carbon atom, a nitrogen atom, or a combination thereof, A is at least one group 12, 13, 14, 15, or 16 element, n is not less than 1 and not more than 4, and m is more than n and not more than 5.

M, X, n, and m have the same meaning as described above.

A is at least one Group 12, 13, 14, 15, or 16 element, is usually a Group A element, typically a Group IIIA element and a Group IVA element, and more particularly may contain at least one selected from the group consisting of Al, Ga, In, Tl, Si, Ge, Sn, Pb, P, As, S, and Cd, and is preferably Al or Si.

The MAX phase has a crystal structure in which a layer constituted by A atoms is located between two layers represented by $M_mX_n$ (each X may have a crystal lattice located in an octahedral array of M). When typically m = n + 1, but not limited thereto, the MAX phase includes repeating units in which each one layer of X atoms is disposed in between adjacent layers of n + 1 layers of M atoms (these are also collectively referred to as an "$M_mX_n$ layer"), and a layer of A atoms ("A atom layer") is disposed as a layer next to the (n + 1)th layer of M atoms. The A atom layer (and optionally a part of the M atoms) is removed by selectively etching (removing and optionally layer-separating) the A atoms (and optionally a part of the M atoms) from the MAX phase.

The MAX phase can be produced by a known method. For example, a TiC powder, a Ti powder, and an Al powder are mixed in a ball mill, and the resulting mixed powder is fired under an Ar atmosphere to obtain a fired body (block-shaped MAX phase). Thereafter, the fired body obtained is crushed by an end mill to obtain a powdery MAX phase for the next step.

. Step (b)

In a step (b), an etching treatment for removing at least a part of the A atoms from the precursor (MAX phase) represented by $M_mAX_n$ is performed. As a result, an etched product in which at least a part of the layer composed of A atoms is removed is obtained while the $M_mX_n$ layer in the precursor is maintained.

Conditions for the etching treatment are not particularly limited, and known conditions can be adopted. The etching may be performed using an etching liquid containing $F^-$. Such an etching liquid may contain hydrofluoric acid, hydrochloric acid, phosphoric acid, or the like as an acid. In one aspect, as the etching liquid, hydrofluoric acid; mixture of hydrofluoric acid and hydrochloric acid; examples thereof include a mixed solution of lithium fluoride and hydrochloric acid, and both may further contain phosphoric acid. As the solvent in the etching liquid, water may be used, and for example, pure water may be used.

The etching treatment may be performed as a slurry by mixing the precursor and the etching liquid.

In one aspect, in the step (b), the intercalation treatment may be performed simultaneously. By coexisting with a metal-containing compound described later in the etching liquid, the etching treatment and the intercalation treatment can be simultaneously performed. In this case, a step (e) described later may be further performed.

When the intercalation treatment is simultaneously performed in the step (b), the content of the metal-containing compound in the total of the precursor, the metal-containing compound, and the etching liquid can be, for example, not less than 0.001 mass% and not more than 10 mass%, further not less than 0.01 mass% and not more than 1 mass%, and particularly not less than 0.1 mass% and not more than 1 mass%.

• Step (c)

In a step (c), the treated product obtained by the etching treatment is cleaned to obtain a cleaned product. By performing the cleaning, the acid and the like used in the etching treatment can be sufficiently removed.

The cleaning may preferably be carried out with water. The amount of water mixed with the etched product and the cleaning method are not particularly limited. For example, stirring, centrifugation, and the like may be performed by adding water. Examples of the stirring method include a stirring method using a handshake, an automatic shaker, a share mixer, a pot mill, or the like. The degree of stirring such as the stirring speed and the stirring time may be adjusted according to the amount, concentration, and the like of the etched product target to be treated. The cleaning with water may be performed once or more, and cleaning with water is preferably performed a plurality of times. For example, specifically, the cleaning with water may be performed by sequentially performing a step (i) (to the treated product or the remaining precipitate obtained in the following (iii)), adding water and stirring, a step (ii) centrifuging the stirred product, and a step (iii) discarding the supernatant liquid after centrifugation, and the steps (i) to (iii) may be repeated within a range of two times or more, for example, 15 times or less.

• Step (d)

In a step (d), an intercalation treatment for obtaining an intercalated product is performed by performing an intercalation treatment on the etched product in a dispersion medium using a metal-containing compound containing a metal ion. As a result, an intercalated product in which the metal ion contained in the metal-containing compound is intercalated between two adjacent $M_mX_n$ layers is obtained.

The metal ion may contain a monovalent metal ion, and examples of the monovalent metal ion include alkali metal ions such as a lithium ion, a sodium ion, and a potassium ion, a copper ion, a silver ion, and a gold ion.

Examples of the metal-containing compound include an ionic compound in which the metal ion and the anion are bonded. Examples of the metal compound include a salt of the metal including an iodide, a phosphate, a sulfide salt including a sulfate, a nitrate, an acetate, and a carboxylate. As the metal ion, a lithium ion is preferable, and as the metal-containing compound, a metal-containing compound containing a lithium ion is preferable, an ionic compound of a lithium ion is more preferable, and one or more of an iodide, a phosphate, and a sulfide salt of a lithium ion is further preferable. When a lithium ion is used as the metal ion, it is considered that water hydrated to the lithium ion has the most negative dielectric constant, and thus it is easy to form a single-layer.

The content of the metal-containing compound in the total of the etched product, the metal-containing compound, and the dispersion medium can be, for example, not less than 0.001 mass% and not more than 10 mass%, further not less than 0.01 mass% and not more than 1 mass%, and particularly not less than 0.1 mass% and not more than 1 mass%. When the content of the metal-containing compound is within the above range, dispersibility in a dispersion medium is good.

A specific method of the intercalation treatment is not particularly limited, and for example, the dispersion medium, the etched product, and the metal-containing compound may be mixed and stirred, or may be left to stand. The examples include stirring at room temperature. Examples of the stirring method include a method using a stirring bar such as a stirrer, a method using a stirring blade, a method using a mixer, a method using a centrifugal device, and the like, and the stirring time can be set according to the production scale of the single-layer/few-layer MXene particles, and can be set, for example, for 12 to 24 hours. The order of mixing the dispersion medium, the etched product, and the metal-containing compound is not particularly limited, but in one aspect, the dispersion medium and the etched product may be mixed, and then the metal-containing compound may be mixed. Typically, the etching liquid after the etching treatment may be used as the dispersion medium.

• Step (e)

In the step (e), the intercalated product obtained by performing the intercalation treatment is subjected to a delamination treatment to obtain a delaminated product. The delamination treatment includes peeling at least a part between two adjacent $M_mX_n$ layers by applying a shear stress to the intercalated product. By the delamination treatment, the MXene particles may become single-layered and few-layered.

Conditions for the delamination treatment are not particularly limited, and the delamination treatment can be performed by a known method. The examples for a method of applying a shear stress to the intercalated product include a method of dispersing the intercalated product in a dispersion medium and stirring the dispersion medium. Examples of the stirring method include stirring using ultrasonic treatment, a handshake, an automatic shaker, or the like. The degree of stirring such as the stirring speed and the stirring time may be adjusted according to the amount, concentration, and the like of the product target to be treated. For example, the slurry after the intercalation is centrifuged to discard the supernatant liquid, then pure water is added to the remaining precipitate, and stirring is performed by, for example, a handshake or an automatic shaker to perform layer separation. The removal of the unpeeled substance includes a step of performing centrifugal separation to discard the supernatant, and then cleaning the remaining precipitate with water. For example, (i) pure water is added to the remaining precipitate after discarding the supernatant and stirred, (ii) centrifugation is performed, and (iii) the supernatant liquid is recovered. This operation of (i) to (iii) is repeated one time or more, preferably two times or more and 10 times or less to obtain a supernatant liquid containing single-layer/few-layer MXene particles as a delaminated product. Alternatively, the supernatant liquid may be centrifuged, and the supernatant liquid after centrifugation may be discarded to obtain a clay containing single-layer/few-layer MXene particles as a delaminated product.

In the method for producing two-dimensional particles, when the intercalation treatment is performed, the cleaning treatment may be further performed at an arbitrary stage after the intercalation treatment, preferably at a stage after the delamination treatment. By performing such a cleaning treatment, the metal ions and the metal-containing compound used for the intercalation can be sufficiently removed. Typically, such a cleaning treatment is performed after the step (e).

In one aspect, the cleaning treatment after the intercalation treatment may be performed in the same manner as in the step (c). In another aspect, after the delaminated product is acid-treated, the acid-treated product may be cleaned in the same manner as in the step (c). In these cases, the etched product in the step (c) may be replaced with a delaminated product or an acid treatment product, and the cleaning treatment may be performed.

The acid treatment can be performed by mixing and stirring the delaminated product and the acid solution. Examples of the acid include inorganic acids such as hydrochloric acid, sulfuric acid, nitric acid, phosphoric acid, perchloric acid, hydroiodic acid, hydrobromic acid, and hydrofluoric acid; organic acids such as acetic acid, citric acid, oxalic acid, benzoic acid, or sorbic acid, and these may be appropriately used. The concentration of the acid in the acid solution may be appropriately adjusted according to the delaminated product. The stirring can be performed using a handshake, an automatic shaker, a share mixer, a pot mill, or the like. The acid treatment may be performed one or more times, and if necessary, an operation of mixing with a fresh acid solution (acid solution not used for the acid treatment) and stirring may be performed within a range of two or more times, for example, 10 times or less.

The intermediate and the target product in the production method described above may be isolated by a commonly used purification method. Examples of such purification methods includes suction filtration; drying such as heat drying, freeze drying, and vacuum drying.

For example, in an adsorption test using human plasma, the two-dimensional particles exhibits an action of adsorbing a disease-causing substance such as an electrolyte (ionic substance) such as $Na^+$, $K^+$, $Mg^{2+}$, $Ca^{2+}$, or P (For example, $PO_4^{3-}$); uremic substances (examples thereof include uremic substances having a molecular weight of 100 or more, and in particular, protein-bound uremic substances such as creatinine and homocysteine; middle molecular type uremic substances such as parathyroid hormone, $\beta$2-microglobulin, and tumor necrosis factor (TNF)-$\alpha$); cytokines (in one aspect, an inflammatory cytokine such as interleukin 18) such as interleukins, interferons, chemokines, hematopoietic factors, cell growth factors, and tumor necrosis factors; proteins such as albumin and M protein; carcinogen; and an inflammatory substance. Therefore, the pharmaceutical composition containing the two-dimensional particles are useful for the treatment or prevention of a disease.

Examples of the diseases include various symptoms associated with renal diseases such as acute kidney disorder (including acute renal failure) and chronic kidney disease (chronic renal failure, including end-stage renal failure), and various symptoms associated with inflammatory diseases such as vascular dementia, inflammatory bowel disease, hepatitis and myocarditis; in particular, various symptoms associated with kidney disorders and various symptoms associated with inflammatory diseases are mentioned, and specifically, uremia; electrolyte imbalance such as hypernatremia, hyperkalemia, hypermagnesemia, hypercalcemia, and hyperphosphatemia; autoimmune diseases; infectious diseases; inflammatory diseases; endocrine and metabolic diseases; circulatory disorders; blood diseases; gastrointestinal diseases; neuropathy; malignant tumors; drug intoxication; vascular dementia, inflammatory bowel disease, hepatitis, and myocarditis.

Furthermore, since the two-dimensional particles can predominantly adsorb the disease-causing substances, it is expected that the two-dimensional particles adsorb the disease-causing substances in vivo and reduce the disease-causing substances in blood by being administered to a subject. In particular, it is expected that the disease-causing substances in the blood can be adsorbed and eliminated from the blood through mutual access between the intestinal tract and the blood vessel while staying in the intestinal tract by oral administration. In addition, it is also expected that reduction of the disease-causing substances in the blood leads to reduction of treatment related to dialysis therapy and reduction of the burden on organs such as the kidney.

The pharmaceutical composition according to the present embodiment can be in various dosage forms depending on usage. Examples of such a dosage form include powder, a granule, a fine granule, dry syrup, a tablet, a capsule, solution, and a sublingual agent, and also include an injection, an ointment, a suppository, and a patch.

The pharmaceutical composition according to the present embodiment can be a pharmaceutical composition further containing two-dimensional particles as an active ingredient and a pharmacologically acceptable additive by a known method according to the dosage form. Examples of such additives include an excipient, a disintegrant, a binder, a lubricant, a diluent, a buffering agent, a tonicity agent, a preservative, a wetting agent, an emulsifying agent, a dispersing agent, a stabilizing agent, and solubilizing agent. The pharmaceutical composition of the present disclosure can be prepared by appropriately mixing the two-dimensional particles and the additive or diluting and dissolving the two-dimensional particles with an additive.

The pharmaceutical composition according to the present embodiment can be administered systemically or locally, orally or parenterally (nasal, pulmonary, intravenous, intrarectal, subcutaneous, muscle, or transdermal). In one aspect, the pharmaceutical composition according to the present embodiment can be administered orally.

When the pharmaceutical composition of the present disclosure is used for treatment, the dose of the two-dimensional particles as an active ingredient thereof is appropriately determined depending on the age, sex, weight, disease, degree of treatment, and the like of the patient. For example, in the case of oral administration, the dose may be appropriately administered once or in several divided doses in the range of about 100 mg to 10 g/body per day of an adult (body weight of 60 kg) as an effective amount.

In addition, the pharmaceutical composition containing the two-dimensional particles can be used for producing a medicine for treating or preventing a disease.

EXAMPLES

The present disclosure is described more specifically with reference to the following examples, but the present disclosure is not limited thereto.

Example 1

Production of MXene

In Examples 1 and 2, (1) preparation of the precursor (MAX), (2) etching of the precursor, and (3) cleaning and drying described in detail below were sequentially performed to prepare two-dimensional particles.

(1) Preparation of precursor (MAX)

TiC powder, Ti powder, and Al powder (all manufactured by Kojundo Chemical Laboratory Co., Ltd.) were placed in a ball mill containing zirconia balls at a molar ratio of 2 : 1 : 1 and mixed for 24 hours. The resulting mixed powder was fired at 1,350°C for 2 hours under an Ar atmosphere. The fired body (block) thus obtained was crushed with an end mill to a maximum size of 40 $\mu$m or less. In this way, $Ti_3AlC_2$ particles were obtained as a precursor (MAX).

(2) Etching of precursor

Using the $Ti_3AlC_2$ particles (powder) prepared by the above method, etching was performed under the following etching conditions to obtain a solid-liquid mixture (slurry) containing a solid component derived from the $Ti_3AlC_2$ powder.

(Etching conditions)

| | |
|---|---|
| • Precursor: | $Ti_3AlC_2$ (Sieving with 45 $\mu$m mesh) |
| • Etching liquid composition: | 50 mass% HF 6 mL, |
| | $H_2O$ 18 mL |
| | HCl (12M) 36 mL |
| • Precursor charge amount: | 3.0 g |
| • Etching container: | 100 mL Iboy |
| • Etching temperature: | 35°C |
| • Etching time: | 24h |
| • Stirrer rotation speed: | 400rpm |

(3) Cleaning and drying

The slurry was divided into two portions, each of which was inserted into two 50 mL centrifuge tubes, centrifuged under the condition of 3,500 G using a centrifuge, and then the supernatant liquid was discarded. An operation of adding 40 mL of pure water to each centrifuge tube, centrifuging again at 3,500 G, and separating and removing the supernatant liquid was repeated 11 times. After final centrifugation, the supernatant liquid was discarded to obtain a $Ti_3C_2T_s$-water medium clay. The obtained clay was dried by freeze drying to obtain a dried powder of two-dimensional particles.

Example 2

In Example 2, (1) the precursor (MAX) was prepared in the same manner as in Example 1, then the following step (2) was performed, and (3) cleaning and drying were performed in the same manner as in Example 1 to prepare two-dimensional particles.
(1) Preparation of precursor: Same as in Example 1
(2) Precursor etching

| | |
|---|---|
| • Precursor: | $Ti_3AlC_2$ (Sieving with 45 $\mu$m mesh) |
| • Etching liquid composition: | 50 mass% HF 5 mL, |
| | $H_2O$ 45 mL |
| • Precursor charge amount: | 3.0 g |
| • Etching container: | 100 mL Iboy |
| • Etching temperature: | 35°C |

(continued)

| • Etching time: | 24h |
| • Stirrer rotation speed: | 400rpm |

(3) Cleaning: Same as in Example 1

Example 3

In Example 3, (1) the precursor (MAX) was prepared in the same manner as in Example 1, then the following step (2) was performed, and (3) cleaning and drying were performed in the same manner as in Example 1 to prepare two-dimensional particles.

(1) Preparation of precursor: Same as in Example 1

(2) Precursor etching

| • Precursor: | $Ti_3AlC_2$ (Sieving with 45 $\mu$m mesh) |
| • Etching liquid composition: | 49 mass% HF aqueous solution 6 mL, |
| | $H_2O$ 9 mL |
| | 85 mass% $H_3PO_4$ aqueous solution 45 mL |
| • Precursor charge amount: | 3.0 g |
| • Etching container: | 100 mL Iboy |
| • Etching temperature: | 35°C |
| • Etching time: | 24h |
| • Stirrer rotation speed: | 400rpm |

(3) Cleaning: Same as in Example 1

Example 4

In Example 4, (1) the precursor (MAX) was prepared in the same manner as in Example 1, then the following step (2) was performed, and (3) cleaning and drying were performed in the same manner as in Example 1 to prepare two-dimensional particles.

(1) Preparation of precursor: Same as in Example 1

(2) Precursor etching

| • Precursor: | $Ti_3AlC_2$ (Sieving with 45 $\mu$m mesh) |
| • Etching liquid composition: | LiF 4.8 g |
| | HCl (9M) 60 mL |
| • Precursor charge amount: | 3.0 g |
| • Etching container: | 100 mL Iboy |
| • Etching temperature: | 35°C |
| • Etching time: | 24h |
| • Stirrer rotation speed: | 400rpm |

(3) Cleaning: Same as in Example 1

Example 5

In Example 5, (1) preparation of the precursor (MAX), (2) etching of the precursor, and (3) cleaning and drying described in detail below were sequentially performed to prepare two-dimensional particles.

(1) Preparation of precursor (MAX)

Into a ball mill containing a zirconia ball, 73 g of Ti powder, 47.2 g of TiN powder, 20.6 g of Al powder, and 9.2 g of C powder (all manufactured by Kojundo Chemical Laboratory Co., Ltd.) were charged, and mixed for 24 hours. The resulting mixed powder was fired at 1,400°C for 2 hours under an Ar atmosphere. The fired body (block) thus obtained was crushed with an end mill to a maximum size of 40 $\mu$m or less. In this way, $Ti_3AlCN$ particles were obtained as a precursor (MAX).

(2) Etching of precursor

Using the $Ti_3AlCN$ particles (powder) prepared by the above method, etching was performed under the following etching conditions to obtain a solid-liquid mixture (slurry) containing a solid component derived from the $Ti_3AlCN$ powder.

(Etching conditions)

| | |
|---|---|
| • Precursor: | $Ti_3AlCN$ (Sieving with 45 $\mu$m mesh) |
| • Etching liquid composition: | 48 mass% HF 6 mL |
| | 38.8 mass% HCl 36 mL |
| | $H_2O$ 18 mL |
| • Precursor charge amount: | 3.0 g |
| • Etching container: | 100 mL Iboy |
| • Etching temperature: | 25°C |
| • Etching time: | 24h |
| • Stirrer rotation speed: | 400rpm |

(3) Cleaning and drying

The slurry was divided into two portions, each of which was inserted into two 50 mL centrifuge tubes, centrifuged under the condition of 3,500 G using a centrifuge, and then the supernatant liquid was discarded. An operation of adding 40 mL of pure water to each centrifuge tube, centrifuging again at 3,500 G, and separating and removing the supernatant liquid was repeated 11 times. After final centrifugation, the supernatant liquid was discarded to obtain a $Ti_3CNT_s$-water medium clay. The obtained clay was dried by freeze drying to obtain a dried powder of two-dimensional particles.

Example 6

In Example 6, (1) preparation of the precursor (MAX), (2) etching of the precursor, and (3) cleaning and drying described in detail below were sequentially performed to prepare two-dimensional particles.

(1) Preparation of precursor (MAX)

Into a ball mill containing zirconia balls, 21.5 g of the V powder, 6.3 g of the Al powder, and 2.3 g of the C powder (all manufactured by Kojundo Chemical Laboratory Co., Ltd.) were charged, and mixed for 24 hours. The resulting mixed powder was fired at 1,550°C for 2 hours under an Ar atmosphere. The fired body (block) thus obtained was crushed with a jaw crusher to a maximum dimension of 45 $\mu$m or less. In this way, $V_2AlC$ particles were obtained as a precursor (MAX).

(2) Etching of precursor

Using the $V_2AlC$ particles (powder) prepared by the above method, etching was performed under the following etching conditions to obtain a solid-liquid mixture (slurry) containing a solid component derived from the $V_2AlC$ powder.

(Etching conditions)

| | |
|---|---|
| • Precursor: | $V_2AlC$ (Sieving with 45 $\mu$m mesh) |
| • Etching liquid composition: | 48 mass% HF 6 mL |
| | 38.8 mass% HCl 24 mL |
| • Precursor charge amount: | 3.0 g |
| • Etching container: | 100 mL Iboy |
| • Etching temperature: | 50°C |
| • Etching time: | 48h |

(continued)

| • Stirrer rotation speed: | 400rpm |
|---|---|

(3) Cleaning and drying

The slurry was divided into two portions, each of which was inserted into two 50 mL centrifuge tubes, centrifuged under the condition of 3,500 G using a centrifuge, and then the supernatant liquid was discarded. An operation of adding 40 mL of pure water to each centrifuge tube, centrifuging again at 3,500 G, and separating and removing the supernatant liquid was repeated 11 times. After final centrifugation, the supernatant liquid was discarded to obtain a $V_2CT_s$-water medium clay. The obtained clay was dried by freeze drying to obtain a dried powder of two-dimensional particles.

Example 7

In Example 7, (1) preparation of the precursor (MAX), (2) etching of the precursor, and (3) cleaning and drying described in detail below were sequentially performed to prepare two-dimensional particles.

(1) Preparation of precursor (MAX)

In a ball mill containing zirconia balls, 105.4 g of Ti powder, 32.7 g of Al powder, and 11.9 g of C powder (all manufactured by Kojundo Chemical Laboratory Co., Ltd.) were charged, and mixed for 24 hours. The resulting mixed powder was fired at 1,550°C for 2 hours under an Ar atmosphere. The fired body (block) thus obtained was crushed with an end mill to a maximum size of 40 $\mu$m or less. In this way, $Ti_2AlC$ particles were obtained as a precursor (MAX).

(2) Etching of precursor

Using the $Ti_2AlC$ particles (powder) prepared by the above method, etching was performed under the following etching conditions to obtain a solid-liquid mixture (slurry) containing a solid component derived from the $Ti_2AlC$ powder.

(Etching conditions)

| • Precursor: | $Ti_2AlC$ (Sieving with 45 $\mu$m mesh) |
|---|---|
| • Etching liquid composition: | 48 mass% HF 2 mL |
| | 38.8 mass% HCl 12 mL |
| | $H_2O$ 6 mL |
| • Precursor charge amount: | 1.0 g |
| • Etching container: | 100 mL Iboy |
| • Etching temperature: | 35°C |
| • Etching time: | 24h |
| • Stirrer rotation speed: | 400rpm |

(3) Cleaning and drying

The slurry was divided into two portions, each of which was inserted into two 50 mL centrifuge tubes, centrifuged under the condition of 3,500 G using a centrifuge, and then the supernatant liquid was discarded. An operation of adding 40 mL of pure water to each centrifuge tube, centrifuging again at 3,500 G, and separating and removing the supernatant liquid was repeated 11 times. After final centrifugation, the supernatant liquid was discarded to obtain a $Ti_2CT_s$-water medium clay. The obtained clay was dried by freeze drying to obtain a dried powder of two-dimensional particles.

Example 8

In Example 8, (1) preparation of the precursor (MAX), (2) etching of the precursor, and (3) cleaning and drying described in detail below were sequentially performed to prepare two-dimensional particles.

(1) Preparation of precursor (MAX)

TiC powder, Ti powder, and Al powder (all manufactured by Kojundo Chemical Laboratory Co., Ltd.) were placed in a ball mill containing zirconia balls at a molar ratio of 2 : 1 : 1 and mixed for 24 hours. The obtained mixed powder was calcined in an Ar atmosphere at 1350°C for 2 hours. The fired body (block) thus obtained was crushed with an end mill to a maximum size of 40 $\mu$m or less. As a result, $Ti_3AlC_2$ particles were obtained as MAX particles.

(2) Etching of precursor

Using the $Ti_3AlC_2$ particles (powder) prepared by the above method, etching was performed under the following etching conditions to obtain a solid-liquid mixture (slurry) containing a solid component derived from the $Ti_3AlC_2$ powder.

(Etching conditions)

| | |
|---|---|
| • Precursor: | $Ti_3AlC_2$ (Sieving with 45 $\mu$m mesh) |
| • Etching liquid composition: | 48%HF 6 mL |
| | $H_2O$ 54 mL |
| • Precursor charge | amount: 3.0 g |
| • Etching container: | 100 mL Iboy |
| • Etching temperature: | 50°C |
| • Etching time: | 24h |
| • Stirrer rotation speed: | 400rpm |

(3) Cleaning and drying

The slurry was divided into two portions, each of which was inserted into two 50 mL centrifuge tubes, centrifuged under the condition of 3500 G using a centrifuge, and then the supernatant liquid was discarded. An operation of adding 40 mL of pure water to the remaining precipitate in each centrifuge tube, centrifuging again at 3500 G, and separating and removing the supernatant liquid was repeated 11 times. After final centrifugation, the supernatant liquid was discarded to obtain a $Ti_3C_2T_x$-water medium clay. The obtained clay was dried by freeze drying to obtain a dried powder of two-dimensional particles.

Example 9

In Example 9, (1) preparation of the precursor (MAX), (2) etching of the precursor, and (3) cleaning and drying described in detail below were sequentially performed to prepare two-dimensional particles.

(1) Preparation of precursor (MAX)

TiC powder, Ti powder, and Al powder (all manufactured by Kojundo Chemical Laboratory Co., Ltd.) were placed in a ball mill containing zirconia balls at a molar ratio of 2 : 1 : 1 and mixed for 24 hours. The obtained mixed powder was calcined in an Ar atmosphere at 1350°C for 2 hours. The fired body (block) thus obtained was crushed with an end mill to a maximum size of 40 $\mu$m or less. As a result, $Ti_3AlC_2$ particles were obtained as MAX particles.

(2) Etching of precursor

Using the $Ti_3AlC_2$ particles (powder) prepared by the above method, etching was performed under the following etching conditions to obtain a solid-liquid mixture (slurry) containing a solid component derived from the $Ti_3AlC_2$ powder.

(Etching conditions)

| | |
|---|---|
| • Precursor: | $Ti_3AlC_2$ (Sieving with 45 $\mu$m mesh) |

(continued)

| | |
|---|---|
| • Etching liquid composition: | 48 mass% HF 6 mL |
| | 38.8 mass% HCl 38 mL |
| | $H_2O$ 16 mL |
| • Precursor charge amount: | 3.0 g |
| • Etching container: | 100 mL Iboy |
| • Etching temperature: | 50°C |
| • Etching time: | 24h |
| • Stirrer rotation speed: | 400rpm |

(3) Cleaning and drying

The slurry was divided into two portions, each of which was inserted into two 50 mL centrifuge tubes, centrifuged under the condition of 3500 G using a centrifuge, and then the supernatant liquid was discarded. An operation of adding 40 mL of pure water to the remaining precipitate in each centrifuge tube, centrifuging again at 3500 G, and separating and removing the supernatant liquid was repeated 11 times. After final centrifugation, the supernatant liquid was discarded to obtain a $Ti_3C_2T_x$-water medium clay. The obtained clay was dried by freeze drying to obtain a dried powder of two-dimensional particles.

Example 10

In Example 10, (1) preparation of a precursor (MAX), (2) etching of the precursor, (3) cleaning, (4) Li intercalation, (5) delamination, (6) modification with an amino group, and drying described in detail below were sequentially performed to prepare two-dimensional particles.

(1) Preparation of precursor (MAX)

TiC powder, Ti powder, and Al powder (all manufactured by Kojundo Chemical Laboratory Co., Ltd.) were placed in a ball mill containing zirconia balls at a molar ratio of 2 : 1 : 1 and mixed for 24 hours. The obtained mixed powder was calcined in an Ar atmosphere at 1350°C for 2 hours. The fired body (block) thus obtained was crushed with an end mill to a maximum size of 40 μm or less. As a result, $Ti_3AlC_2$ particles were obtained as MAX particles.

(2) Etching of precursor

Using the $Ti_3AlC_2$ particles (powder) prepared by the above method, etching was performed under the following etching conditions to obtain a solid-liquid mixture (slurry) containing a solid component derived from the $Ti_3AlC_2$ powder.

(Etching conditions)

| | |
|---|---|
| • Precursor: | $Ti_3AlC_2$ (Sieving with 45 μm mesh) |
| • Etching liquid composition: | 48 mass% HF 2 mL |
| | 38.8 mass% HCl 38 mL |
| | $H_2O$ 16 mL |
| • Precursor charge amount: | 3.0 g |
| • Etching container: | 100 mL Iboy |
| • Etching temperature: | 50°C |
| • Etching time: | 24h |
| • Stirrer rotation speed: | 400rpm |

(3) Cleaning and drying

The slurry was divided into two portions, each of which was inserted into two 50 mL centrifuge tubes, centrifuged under the condition of 3500 G using a centrifuge, and then the supernatant liquid was discarded. An operation of adding 40 mL of pure water to the remaining precipitate in each centrifuge tube, centrifuging again at 3500 G, and separating and removing the supernatant liquid was repeated 11 times. After final centrifugation, the supernatant liquid was discarded to obtain a $Ti_3C_2T_x$-water medium clay.

(4) Li intercalation

The $Ti_3C_2T_x$-water medium clay prepared by the above method was stirred at not less than 20°C and not more than 25°C for 12 hours using LiCl as a Li-containing compound to perform Li intercalation according to the following conditions.

(Conditions of Li intercalation)

- $Ti_3C_2T_x$-water medium clay (MXene after cleaning):    Solid content 0.75 g
- LiCl:    0.75 g
- Intercalation container:    100 mL Iboy
- Temperature:    Not less than 20°C and not more than 25°C (room temperature)
- Time:    10h
- Stirrer rotation speed:    800rpm

(5) Delamination

(i) To the $Ti_3C_2T_x$-water medium clay, 40 mL of pure water was added, then the mixture was stirred with a shaker for 15 minutes, (ii) the mixture was centrifuged at 3500 G, and (iii) the supernatant liquid was recovered as a single-layer MXene-containing liquid. The operations (i) to (iii) were repeated four times in total to obtain a single-layer MXene-containing supernatant liquid. Further, this supernatant liquid was centrifuged under the conditions of 4,300 G and 2 hours using a centrifuge, and then the supernatant liquid was discarded to obtain a single-layer/few-layer MXene-containing clay as a single-layer/few-layer MXene-containing sample.

(6) Modification with amino group and drying

To 0.34 g of single-layer/few-layer MXene-containing clay, 20 mg of 6-aminohexyl phosphate hydrochloride and 5 mL of pure water were added, and the mixture was stirred at room temperature at a stirrer rotation speed of 800 rpm for 2 hours. The obtained slurry was centrifuged at a rotation speed of 8,000 rpm for 8 minutes, and the supernatant liquid was discarded. Pure water having the same volume as the discarded supernatant liquid was added, and centrifuged at a rotation speed of 8,000 rpm for 8 minutes. Discard of the supernatant liquid, addition of pure water having the same volume as that of the discarded supernatant liquid, and centrifugation at a rotation speed of 8,000 rpm for 8 minutes were repeated once. Subsequently, the supernatant liquid was discarded, and ethanol having the same volume as that of the discarded supernatant liquid was added and dispersed, and then naturally dried to obtain $Ti_3C_2$ particles (powder) modified with an amino group.

Comparative Example 1

The spherical adsorption charcoal ("KREMEZIN Tablets 500 mg", manufactured by KUREHA CORPORATION) was powdered using a mortar and subjected to adsorption evaluation.

Comparative Example 2

An adsorption type blood purifier ("Lixelle", manufactured by KANEKA CORPORATION) was disassembled, and the extracted adsorbing body was subjected to adsorption evaluation.

Comparative Example 3

A medicinal charcoal (manufactured by Nichi-Iko Pharmaceutical Co., Ltd.) was subjected to adsorption evaluation.

Adsorption evaluation method

Human plasma collected from a healthy subject, two-dimensional particles of Example, and spherical adsorption charcoal, adsorption type blood purifier, or medicinal charcoal of Comparative Example (hereinafter, these are also collectively referred to as "adsorbent material") were weighed in a 50 mL centrifuge tube, and shaken and stirred for 60 minutes using a constant temperature shaker (TAITEC BR-33FL) set at 37°C. Thereafter, several mL of the mixed solution was sampled, the adsorbent material was separated with a syringe filter (Millex diameter for Merck Millipore sterilization: 33 mm, hole diameter: 0.45 $\mu$m) having a hole diameter of 0.45 $\mu$m, and then component analysis was performed. In Examples 1 to 4 and Comparative Example 1 to 3, the human plasma amount was 10 mL and the adsorbent material amount was 0.6 g, in Example 5 to 8 and Example 10, the human plasma amount was 4 mL and the adsorbent material amount was 0.2 g, and in Example 9, the human plasma amount was 4 mL and the adsorbent material amount was 0.2 g.

In Examples 1 to 4 and Comparative Examples 1 to 3, component analysis was performed for Na, P, K, Urea, Creatinine, Homocysteine, Folic acid, Parathyroid hormone (Prathyroid hormone), $\beta$2-microglobulin, Interleukin-18, Trypsin, TNF-$\alpha$ (Tumor necrosis factor-$\alpha$), $\alpha$-amylase, and Albumin.

In Examples 5 to 10, component analysis was performed for Albumin, Chlor (Cl), serum urea nitrogen (BUN), $\alpha$-amylase (AMY), lipase, Na, K, inorganic phosphorus (IP), and $\beta$2-microglobulin.

The measurement of Na, K and Cl was performed by an electrode method; the measurement of P, inorganic phosphorus, $\alpha$-amylase, lipase, and creatinine was performed by an enzymatic method; the measurement of urea and serum urea nitrogen was performed by a urease/GLDH/UV method; the measurement of homocysteine was performed by HPLC method; the measurement of folic acid was performed by the CLIA method; the measurement of parathyroid hormone was performed by the ECLIA method; the measurement of $\beta$2-microglobulin was performed by a latex agglutination method; the measurement of interleukin -18, trypsin, and TNF-$\alpha$ was performed by an EIA method; the measurement of albumin was performed by a colorimetric method (BGC method).

Note that the same treatment was also performed on the sample containing no adsorbent material and containing only human plasma in the centrifuge tube, and the sample was used as a baseline of component concentration. The concentration of the component A contained in the human plasma after the adsorption test was defined as $C_{A1}$, and the concentration of the component A at the baseline was defined as $C_{A0}$, and the adsorption removal rate was calculated based on the following formula. When the adsorption removal rate was less than 0% by mass, the adsorption removal rate was set to 0% by mass.

$$\text{Adsorption removal rate (mass\%)} = (C_{A0} - C_{A1})/C_{A0} \times 100$$

The results are shown in Tables 1 and 2.

Table 1

| Components | molecular weight | Example 1 | Example 2 | Example 3 | Example 4 | Comparative Example 1 | Comparative Example 2 | Comparative Example 3 |
|---|---|---|---|---|---|---|---|---|
| Na | 23 | 21 | 22 | 21 | 20 | 0 | 0 | 0 |
| P | 31 | 44 | 46 | 40 | 42 | 0 | 0 | 0 |
| K | 39 | 29 | 29 | 29 | 28 | 0 | 0 | 0 |
| Urea | 60 | 0 | 0 | 2 | 1 | 13 | 0 | 9 |
| Creatinine | 113 | 92 | 91 | 90 | 90 | 91 | 0 | 90 |
| Homocysteine | 135 | 70 | 72 | 68 | 70 | 0 | 0 | 86 |
| Folic acid | 441 | 87 | 80 | 81 | 75 | 68 | 0 | 42 |
| Parathyroid hormone | 9,500 | 73 | 75 | 70 | 70 | 0 | 0 | 35 |
| β2-microglobulin | 11,818 | 50 | 55 | 46 | 52 | 0 | 99 | 5 |
| Interleukin-18 | 18,000 | 29 | 33 | 28 | 27 | 0 | 0 | 0 |
| Trypsin | 24,000 | 26 | 25 | 22 | 24 | 0 | 0 | 0 |
| Tumor necrosis factor-a | 25,000 | 50 | 39 | 28 | 32 | 0 | 0 | 0 |
| α-amylase | 54,000 | 26 | 22 | 22 | 18 | 0 | 0 | 0 |
| Albumin | 66,000 | 11 | 10 | 8 | 11 | 0 | 0 | 0 |

Table 2

| Components | molecular weight | Example 5 | Example 6 | Example 7 | Example 8 | Example 9 | Example 10 |
|---|---|---|---|---|---|---|---|
| Albumin | 66,000 | 3 | 3 | 0 | 0 | 3 | 0 |
| Cl | 35 | 95 | 13 | 95 | 55 | 30 | 0 |
| BUN | 60 | 0 | 0 | 0 | 0 | 0 | 0 |
| AMY | 54,000 | 0 | 0 | 16 | 0 | 0 | 0 |
| Lipase | 45,000 | 0 | 0 | 0 | 0 | 0 | 0 |
| Na | 23 | 19 | 6 | 26 | 6 | 8 | 1 |
| K | 39 | 32 | 21 | 46 | 10 | 11 | 3 |
| IP | 31 | 56 | 0 | 58 | 7 | 42 | 33 |
| β2-microglobulin | 11,818 | 8 | 0 | 75 | 0 | 0 | 25 |

It was confirmed that the two-dimensional particles of Example 1 to 4 exhibited an action of adsorbing a disease-causing substance, particularly an electrolyte (ionic substance), a uremic substance, an inflammatory cytokine, and other proteins. Therefore, the pharmaceutical composition containing the two-dimensional particles may be useful for treatment of a disease.

In addition, it was confirmed that the two-dimensional particles of Examples 5 to 10 exhibited an action of adsorbing a disease-causing substance, particularly an electrolyte (ionic substance), a uremic substance, and other proteins. Therefore, the pharmaceutical composition containing the two-dimensional particles may be useful for treatment of a disease.

Animal experiment 1 Administration experiment to renal failure model rat

Five-sixths of the kidneys were removed by surgery, and the two-dimensional particles (MXene) produced in Example 1 were administered (10% in the high dose group and 5% in the low dose group with respect to food consumption) to rats artificially brought into a state of renal failure for 28 days. Urea nitrogen (BUN) and serum creatinine (Cr) of the rats were compared to confirm the treatment effect on chronic renal failure. As comparative examples, a Sham group in which only laparotomy was performed and nephrectomy was not performed, and a 5/6 nephrectomy group in which nephrectomy was performed but MXene administration was not performed were prepared. Lower urea nitrogen (BUN) and serum creatinine (Cr) values indicate better renal function.

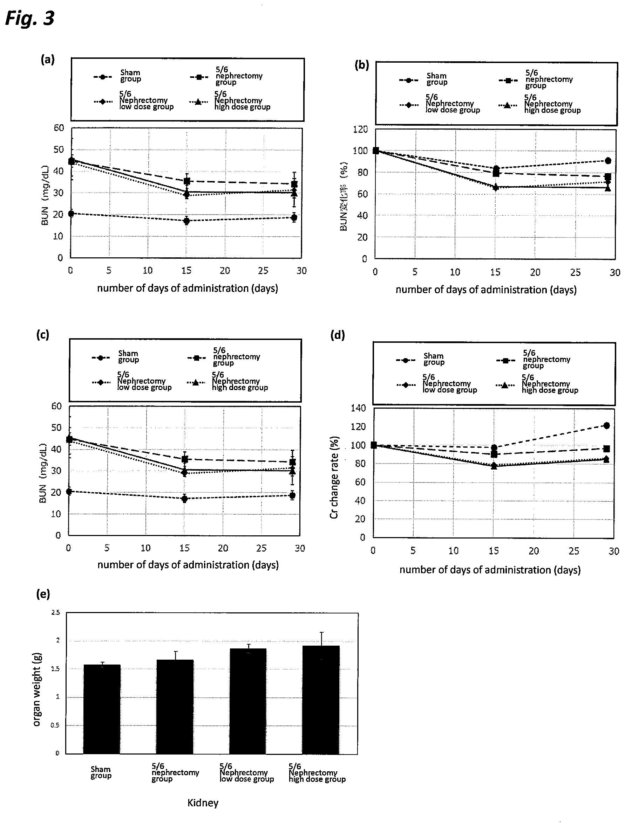

As shown in Figs. 3(a) and 3(b), it was confirmed that the numerical value of urea nitrogen (BUN) decreased at the time point of the number of administration days of 15 in all of the 5/6 nephrectomy group, the high dose group, and the low dose group, and maintained a low value even after the number of administration days elapsed. In addition, a more remarkable decrease was shown in the high dose group and the low dose group, which are MXene administration groups. The decrease in the value of urea nitrogen (BUN) was considered to be due to compensatory hypertrophy, and as shown in Fig. 3(e), more remarkable hypertrophy of the kidneys was confirmed in the high dose group and the low dose group to which MXene was administered. Although the Sham group was performed to confirm the influence of stress in the laparotomy on the renal function, it was confirmed that there was no noticeable influence.

As shown in Figs. 3(c) and 3(d), it was confirmed that the numerical value of serum creatinine (Cr) decreased at the time point of the number of days of administration 15 in any of the 5/6 nephrectomy group, the high dose group, and the low dose group, and maintained a low value even after the number of days of administration elapsed. In addition, a more remarkable decrease was shown in the high dose group and the low dose group, which are MXene administration groups. The decrease in the value of serum creatinine (Cr) was considered to be due to compensatory hypertrophy, and as shown in Fig. 3(e), more remarkable hypertrophy of the kidneys was confirmed in the high dose group and the low dose group to which MXene was administered. Although the Sham group was performed to confirm the influence of stress in the laparotomy on the renal function, it was confirmed that there was no noticeable influence.

Therefore, it can be said that the uremic substance was removed from the body by MXene administration, the symptoms associated with the kidney disorder were reduced, and the renal function was recovered.

Animal experiment 2 Administration experiment to hypertensive mouse

Hypertension causes brain capillaries to become inflamed and produce chronic hypoperfusion. The symptom that the cognitive function is lowered by this is called vascular dementia. In animal experiment 2, MXene was administered to a mouse having genetically high blood pressure to remove an inflammatory substance in the body, and it was confirmed whether recovery of cognitive function was expected. The administration period was 56 days, and the dose was 8% of the food consumption. After the end of the administration period, blood pressure fluctuation and cognitive behavior analysis (Novel Object Recognition Test) were performed. As a comparative example, a Ctl group to which MXene was not administered was also prepared.

In the cognitive behavior analysis (Novel Object Recognition Test), a black box having a vertical width of 400 mm, a horizontal width of 400 mm, and a height of 400 mm was used. On days 1 and 2, mice were left in the box for 10 minutes to acclimatize. On day 3, two identical substances (old substances) were placed in cages, and the mice were left for 10 minutes. On day 4, one of the objects was changed to a new substance, and the mice were left for 10 minutes. Time for searching both objects was measured, and the ratio of the search time for a new substance to the total search time (Times of Entries Discrimination Index) was calculated according to the following equation:

$$\text{Times of Entries Discrimination Index} = [n/(n + f)] - 0.5$$

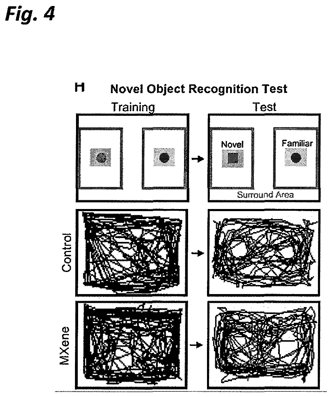

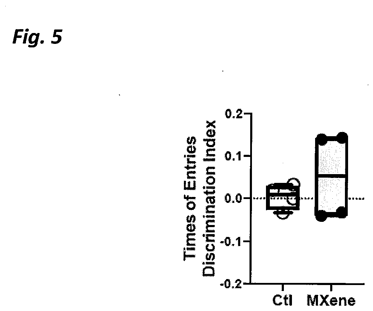

wherein n represents the number of times of touching the new object, and f represents the number of times of touching the conventional object.
and used as an index of memory learning ability. The mouse action region is shown in Fig. 4, and the Times of Entries Discrimination Index is shown in Fig. 5.

Although a decrease in blood pressure due to MXene administration was not observed, in the Novel Object Recognition Test, it was confirmed that the Times of Entries Discrimination Index was higher in the MXene administration group and was a positive number. This indicates that the number of times of interest in and approaching the new substance is higher than that of the old substance. That is, it is suggested that a new substance has been recognized as a new substance, and the cognitive function has recovered.

From this, it can be said that the inflammatory substance was removed by MXene administration, and the symptoms associated with the vascular disorder were reduced.

The present disclosure comprises the following:

[1] A pharmaceutical composition for adsorbing a disease-causing substance by oral administration, the pharmaceutical composition comprising two-dimensional particles having one or plural layers,
the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.
[2] The pharmaceutical composition according to [1], wherein the disease-causing substance comprises a uremic substance having a molecular weight of 100 or more.
[3] The pharmaceutical composition according to [2], wherein the uremic substance having a molecular weight of 100 or more comprises β2-microglobulin.
[4] The pharmaceutical composition according to any one of [1] to [3], wherein the disease-causing substance comprises a cytokine.
[5] The pharmaceutical composition according to any one of [1] to [4], wherein the cytokine comprises at least one selected from the group consisting of interleukins, interferons, a chemokine, a hematopoietic factor, a cell growth factor, and a tumor necrosis factor.
[6] The pharmaceutical composition according to any one of [1] to [5], wherein the disease-causing substance comprises an electrolyte.
[7] The pharmaceutical composition according to any one of [1] to [6], wherein the disease-causing substance comprises at least one selected from the group consisting of $Na^+$, $K^+$, $Mg^{2+}$, $Ca^{2+}$, and $PO_4^{3-}$.
[8] An adsorption method comprising administering an effective amount of two-dimensional particles having one or plural layers to a subject to adsorb a disease-causing substance in vivo,
the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

[9] A method for treating or preventing various symptoms associated with renal failure,

the method comprising administering an effective amount of two-dimensional particles having one or plural layers to a subject,
the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

[10] The method according to [9], wherein the various symptoms associated with renal failure are uremia, hypernatremia, hyperkalemia, hypermagnesemia, hypercalcemia, or hyperphosphatemia.

[11] A method for treating or preventing an inflammatory disease,

the method comprising administering an effective amount of two-dimensional particles having one or plural layers to a subject,
the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

[12] The method according to [11], wherein the inflammatory disease comprises inflammatory bowel disease, vascular dementia, hepatitis, myocarditis, and the like.

REFERENCE SIGNS LIST

1a, 1b Layer body ($M_mX_n$ layer)
3a, 5a, 3b, 5b Modifier or terminal T
7a, 7b MXene layer
10, 10a, 10b MXene particles (two-dimensional particles of layered material)

**Claims**

1. A pharmaceutical composition for adsorbing a disease-causing substance by oral administration, the pharmaceutical composition comprising two-dimensional particles having one or plural layers,

the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

2. The pharmaceutical composition according to claim 1, wherein the disease-causing substance comprises a uremic substance having a molecular weight of 100 or more.

3. The pharmaceutical composition according to claim 2, wherein the uremic substance having a molecular weight of 100 or more comprises $\beta$2-microglobulin.

4. The pharmaceutical composition according to any one of claims 1 to 3, wherein the disease-causing substance comprises a cytokine.

5. The pharmaceutical composition according to any one of claims 1 to 4, wherein the cytokine comprises at least one selected from the group consisting of interleukins, interferons, a chemokine, a hematopoietic factor, a cell growth factor, and a tumor necrosis factor.

6. The pharmaceutical composition according to any one of claims 1 to 5, wherein the disease-causing substance comprises an electrolyte.

7. The pharmaceutical composition according to any one of claims 1 to 6, wherein the disease-causing substance comprises at least one selected from the group consisting of $Na^+$, $K^+$, $Mg^{2+}$, $Ca^{2+}$, and $PO_4^{3-}$.

8. An adsorption method comprising administering an effective amount of two-dimensional particles having one or plural layers to a subject to adsorb a disease-causing substance in vivo,

the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

9. A method for treating or preventing various symptoms associated with renal failure,

the method comprising administering an effective amount of two-dimensional particles having one or plural layers to a subject,
the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

10. The method according to claim 9, wherein the various symptoms associated with renal failure are uremia, hypernatremia, hyperkalemia, hypermagnesemia, hypercalcemia, or hyperphosphatemia.

11. A method for treating or preventing an inflammatory disease,

the method comprising administering an effective amount of two-dimensional particles having one or plural layers to a subject,
the layers comprising a layer body represented by a formula below:

$$M_mX_n$$

wherein M is at least one metal of Group 3, 4, 5, 6, or 7, X is a carbon atom, a nitrogen atom, or a combination thereof, n is not less than 1 and not more than 4, m is more than n but not more than 5, and a modifier or terminal T existing on a surface of the layer body, wherein T is at least one selected from the group consisting of a hydroxyl group, a fluorine atom, a chlorine atom, an oxygen atom, and a hydrogen atom.

12. The method according to claim 11, wherein the inflammatory disease comprises inflammatory bowel disease, vascular dementia, hepatitis, myocarditis, and the like.

Fig. 1 (a)

Fig. 1 (b)

*Fig. 2*

## Fig. 3

*Fig. 4*

H   Novel Object Recognition Test

*Fig. 5*

## INTERNATIONAL SEARCH REPORT

| International application No. |
| --- |
| **PCT/JP2023/015821** |

### A. CLASSIFICATION OF SUBJECT MATTER

*A61K 33/00*(2006.01)i; *A61K 9/14*(2006.01)i; *A61M 1/16*(2006.01)i; *A61M 1/36*(2006.01)i; *A61P 7/00*(2006.01)i;
*A61P 13/12*(2006.01)i; *A61P 39/02*(2006.01)i; *B01D 15/00*(2006.01)i; *B01D 69/10*(2006.01)i; *B01D 69/12*(2006.01)i;
*B01D 71/02*(2006.01)i; *B01J 20/02*(2006.01)i; *B01J 20/20*(2006.01)i; *B01J 20/30*(2006.01)i
FI:    A61K33/00; B01J20/20 D; A61P13/12; A61P39/02; A61P7/00; A61K9/14; B01J20/30; A61M1/36 165; B01D69/12;
       B01D69/10; B01D15/00 101Z; B01D15/00 M; B01D71/02; A61M1/16 103; A61M1/16 101; B01J20/02 B

According to International Patent Classification (IPC) or to both national classification and IPC

### B. FIELDS SEARCHED

Minimum documentation searched (classification system followed by classification symbols)

A61K33/00; A61K9/14; A61M1/16; A61M1/36; A61P7/00; A61P13/12; A61P39/02; B01D15/00; B01D69/10; B01D69/12;
B01D71/02; B01J20/02; B01J20/20; B01J20/30

Documentation searched other than minimum documentation to the extent that such documents are included in the fields searched

Published examined utility model applications of Japan 1922-1996
Published unexamined utility model applications of Japan 1971-2023
Registered utility model specifications of Japan 1996-2023
Published registered utility model applications of Japan 1994-2023

Electronic data base consulted during the international search (name of data base and, where practicable, search terms used)

JSTPlus/JMEDPlus/JST7580 (JDreamIII); CAplus/MEDLINE/EMBASE/BIOSIS (STN)

### C. DOCUMENTS CONSIDERED TO BE RELEVANT

| Category* | Citation of document, with indication, where appropriate, of the relevant passages | Relevant to claim No. |
| --- | --- | --- |
| Y | ZHAO, Qi et al. Adsorption of Uremic Toxins Using Ti3C2Tx MXene for Dialysate Regeneration. ACS Nano. 2020, vol. 14, pp. 11787-11798<br>abstract, fig. 1-2 | 1-11 |
| Y | OZULUMBA, Tochukwu et al. Moderating cellular inflammation using 2-dimensional titanium carbide MXene and graphene variants. Biomater. Sci. 2021, vol. 9, pp. 1805-1815<br>abstract, fig. 1, 8-9, table 1 | 1-11 |
| Y | WANG, Tianyi et al. Ultraefficiently Calming Cytokine Storm Using Ti3C2Tx MXene. Small Methods. 2021, vol. 5, 2001108, pp. 1-10<br>abstract, fig. 1, 5 | 1-11 |

☑ Further documents are listed in the continuation of Box C.    ☑ See patent family annex.

| * | Special categories of cited documents: | "T" | later document published after the international filing date or priority date and not in conflict with the application but cited to understand the principle or theory underlying the invention |
| --- | --- | --- | --- |
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
| --- | --- |
| **10 July 2023** | **18 July 2023** |

| Name and mailing address of the ISA/JP | Authorized officer |
| --- | --- |
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

| | International application No. |
|---|---|
| | **PCT/JP2023/015821** |

**C.    DOCUMENTS CONSIDERED TO BE RELEVANT**

| Category* | Citation of document, with indication, where appropriate, of the relevant passages | Relevant to claim No. |
|---|---|---|
| Y | 山本　卓, 大島賞受賞講演2 尿毒症物質に着目した慢性腎臓病関連疾患の 病態解明と 治療, 日腎会誌, 2016, vol. 58(8), pp. 1250-1254<br>　　p. 1252, right column, line 12 to p. 1253, left column, line 23, (YAMAMOTO, Suguru. The Japanese journal of nephrology.), non-official translation (Oshima Award Lecture 2: Pathology elucidation and treatment of chronic kidney disease with focus on uremic substances) | 1-11 |
| Y | DURANTON, Flore et al. Normal and Pathologic Concentrations of Uremic Toxins. J. Am. Soc. Nephrol. 2012, vol. 23, pp. 1258-1270<br>　　abstract, tables 1-7 | 1-11 |
| Y | SHARMA, Geetu et al. Calorimetric Study of Alkali Metal Ion (K+, Na+, Li+) Exchange in a Clay-Like MXene. J. Phys. Chem. C. 2017, vol. 121, pp. 15145-15153<br>　　abstract | 1-11 |
| X<br><br><br>Y | ZHAO, Xing et al. Redox-Mediated Artificial Non-Enzymatic Antioxidant MXene Nanoplatforms for Acute Kidney Injury Alleviation. Adv. Sci. 2021, vol. 8, 2101498, pp. 1-13<br>　　abstract, fig. 1, 2, 5, p. 1, right column, lines 2-15, p. 5, left column, lines 15-45 | 8-9, 11<br><br><br>10 |
| Y | PIENIAZEK, Anna et al. Uremic Toxins and Their Relation with Oxidative Stress Induced in Patients with CKD. Int. J. Mol. Sci. 2021, vol. 22, 6196, pp. 1-21<br>　　abstract | 10 |
| X | XIONG, Wenfang et al. Ultrathin niobium carbide alleviates colitis through absorbing ROS. Materials & Design. 23 December 2021, vol. 213, 110351, pp. 1-10<br>　　abstract, p. 4, right column, lines 23-33, fig. 5-6 | 12 |
| P, X | HOU, Linqian et al. Orally administered titanium carbide nanosheets as anti-inflammatory therapy for colitis. Theranostics. 09 May 2022, vol. 12(8), pp. 3834-3846<br>　　abstract, scheme 1, fig. 4-7 | 12 |
| P, X | CN 115531411 A (XIANGYA HOSPITAL CENTRAL SOUTH UNIVERSITY) 30 December 2022 (2022-12-30)<br>　　claims, examples | 12 |

Form PCT/ISA/210 (second sheet) (January 2015)

**INTERNATIONAL SEARCH REPORT**
Information on patent family members

| International application No. |
| --- |
| **PCT/JP2023/015821** |

| Patent document cited in search report | Publication date (day/month/year) | Patent family member(s) | Publication date (day/month/year) |
| --- | --- | --- | --- |
| CN 115531411 A | 30 December 2022 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Non-patent literature cited in the description**

- Lixelle. KANEKA CORPORATION, January 2017
- medicinal charcoal. Nichi-Iko Pharmaceutical Co., Ltd., January 2019
- KREMEZIN Tablets 500 mg. Mitsubishi Tanabe Pharma Corporation, September 2018
- **YOSHITERU HONDA et al.** Study on Adsorption Property of Spherical Adsorption Charcoal (KREMEZIN Active Material). Hospital Pharmaceutical Co., Ltd., 1997, vol. 23, 219-224
- Kalimate Powder'', ''Kalimate Dry Syrup 92.59%'', ''Kalimate Oral Solution 20%. Kowa Company, Ltd., September 2020
- PHOSBLOCK Tablets 250 mg. Kyowa Kirin Co., Ltd., November 2020